# EUROPÄISCHE PATENTSCHRIFT

(11) **EP 0 987 256 B1**
(45) Veröffentlichungstag und Bekanntmachung des Hinweises auf die Patenterteilung: **17.10.2001**
(21) Anmeldenummer: 99117098.6
(22) Anmeldetag: 31.07.1998
(51) Int. Cl.: C07D 261/18, A61K 31/42

(54) **Kristallform von N-(4-Trifluormethylphenyl)-5-methylisoxazol-4-carbonsäureamid**
Crystal form of N-(4-trifluoromethylphenyl)-5-methylisoxazole-4-carboxamid
Forme cristalline de N-(4-trifluorométhylphényl)-5-méthylisoxazole-4-carboxamid

(30) Priorität: 08.08.1997 DE 19734438; 17.12.1997 DE 19756093
(43) Veröffentlichungstag der Anmeldung: 22.03.2000
(62) Teilanmeldung aus: 98114373.8
(73) Patentinhaber: Aventis Pharma Deutschland GmbH, 65929 Frankfurt am Main (DE)
(72) Erfinder: Faasch, Holger, Dr., 55232 Alzey (DE); Hedtmann, Udo, Dr., 60433 Frankfurt (DE); Westenfelder, Uwe, Dr., 65835 Liederbach (DE); Paulus, Erich, Dr., 65817 Eppstein (DE)

(56) Entgegenhaltungen:
- EP-A- 0 013 376
- EP-A- 0 617 959
- WO-A-91/17748
- DE-A- 2 524 959
- DE-A- 4 127 737

## Beschreibung

Die Kristallmodifikation (Modifikation 2) der Verbindung der Formel I 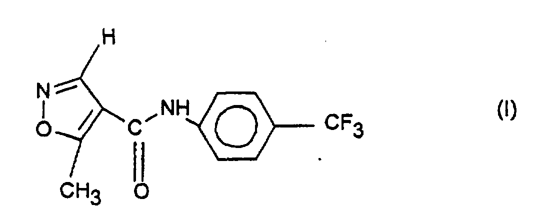 weist im Röntgenbeugungsdiagramm in Transmission mit fokusierendem Debye-Scherrer-Strahlengang und Cu-K_{α1}-Strahlung Linien bei folgenden Beugungswinkeln 2Theta (°) auf:
- Linien starker Intensität:: 10,65; 14,20; 14,80; 16,10; 21,70; 23,15; 24,40; 24,85;
25,50; 25,85; 26,90; 29,85
- Linien mittlerer Intensität:: 7,40; 9,80; 13,10; 15,45; 16,80; 20,70; 21,45; 22,80;
23,85; 27,25; 28,95

Das mit Cu-K_{α1}-Strahlung aufgenommene Röntgenbeugungsdiagramm der Modifikation 2 ist in Figur 1 dargestellt. Zur Aufnahme des Diagramms wurden das Zweikreisdiffraktometer STADI P der Firma Stoe (Darmstadt, Deutschland) oder das rechnergestützte Einkristalldiffraktometer R3 m/V der Firma Siemens (eingesetzte Strahlung Mo K_{α} ) verwendet.

Das Infrarot-Spektrum der Modifikation 2 der Verbindung der Formel I (1 mg in 300 mg KBr) aufgenommen mit einem Infrarot-Spektrophotometer zeigt die folgenden Hauptbanden (Einheit cm⁻¹):

| | | | |
|---|---|---|---|
| 1321 | 1481 | 672 | 3201 |
| 1607 | 3355 | 763 | 701 |
| 1109 | 1264 | 908 | 948 |
| 1065 | 1384 | 754 | 511 |
| 1536 | 1361 | 592 | 733 |
| 1663 | 852 | 427 | 960 |
| 1241 | 1014 | 3111 | 1779 |
| 1410 | 3297 | 3065 | 1811 |
| 1160 | 877 | 3221 | 484 |
| 1691 | 940 | 974 | 3442 |
| 831 | 3274 | 3129 | 3434 |
| 1188 | 894 | 628 | |

Die angegebenen Wellenzahlen sind in ansteigender Intensität angeordnet. Das Infrarot-Spektrum der Modifikation 2 der Verbindung der Formel I ist ferner in Figur 3 dargestellt, wobei in der Ordinate die Transmission in % und auf der Abszisse die Wellenzahl in cm⁻¹ angegeben wurde.

Die Verbindung der Formel I kristallisiert in der Modifikation 2 in der Raumgruppe P2₁/c mit 8 Molekülen in der Elementarzelle. Die Moleküle der Verbindung der Formel I liegen als Dimere vor, die aus den Einzelmolekülen durch Bildung einer-C=O...HN-Wasserstoffbrückenbindung (2,938 Å) hervorgehen, wobei die zwei Molekülebenen praktisch senkrecht zueinander stehen (91,2°). Die beiden Moleküle haben eine stark unterschiedliche Konformation. Die Winkel der Fünf- und Sechsringe mit der zentralen Carbonylgruppe betragen 5,4 ° und 2,1 ° beziehungsweise 23,4 ° und 23,1°. Die letztere Verdrillung schafft die sterische Voraussetzungen dafür, daß die Wasserstoffbrückenbindung zwischen den beiden Molekülen möglich wird.

Die Verbindung der Formel I ist an sich bekannt und wird auch als Leflunomid (HWA 486) bezeichnet. Sie kann auf die in EP 0 013 376 beschriebene Weise erhalten werden. Die durch Umkristallisation aus beispielsweise Toluol hergestellten Kristalle fallen in der Kristallmodifikation 1 an. Das Röntgenbeugungsdiagramm (Cu-K_{α1}-Strahlung) der Modifikation 1 ist in Figur 2 wiedergegeben und weist charakteristische Linien bei folgenden Beugungswinkeln 2 Theta (°) auf:
- Linien starker Intensität:: 16,70; 18,90; 23,00; 23,65; 29,05
- Linien mittlerer Intensität:: 8,35; 12,65; 15,00; 15,30; 18,35; 21,25; 22,15; 24,10;
24,65; 25,45; 26,65; 27,40; 28,00; 28,30

Die Verbindung der Formel I kristallisiert in der Modifikation 1 in der Raumgruppe P2₁/c mit 4 Molekülen in der Elementarzelle. Das Molekül ist im wesentlichen planar. Der Winkel zwischen den planaren Atomgruppen ist kleiner als 2,4°. Die Moleküle sind im Kristall in Stapeln angeordnet. Die Moleküle liegen in Stapeln nebeneinander und sind in einer antiparallelen Art angeordnet. Sehr schwache Wasserstoffbrückenbindungen verbinden die Dimere im Kristallverband (NH ... N: 3,1444 Å). Die C=O-Gruppe ist an keiner Wasserstoffbrückenbindung beteiligt.

Die Erfindung betrifft Verfahren zur Herstellung der Verbindung der Formel I in der Modifikation 1. Durch die Verfahren können auch Mischungen, enthaltend die Modifikation 1 und 2, gezielt in die Modifikation 1 überführt werden. Dazu werden beispielsweise Kristalle der Modifikation 2 oder Mischungen von Modifikation 1 und 2 in einem Lösungsmittel gelöst. Als Lösungsmittel geeignet sind beispielsweise mit Wasser mischbare Lösungsmittel wie (C₁-C₄)-Alkohole, z.B. Methanol, Ethanol, Propanol, Isopropanol, Butanol oder Isobutanol, aber auch Ketone wie Aceton oder Methylethylketon. Bewährt haben sich auch Mischungen von organischen Lösungsmitteln mit Wasser, beispielsweise von etwa 40 % bis 90 % Isopropanol. Der Lösevorgang wird vorzugsweise bei erhöhter Temperatur bis zum Siedepunkt des jeweiligen Lösungsmittels durchgeführt. Die heiße Lösung wird für einige Zeit auf Siedetemperatur gehalten um eine vollständige Lösung der Verbindung der Formel I sicherzustellen. Danach wird die filtrierte Lösung so langsam abgekühlt, daß sich nur Kristalle der Modifikation 1 bilden. Bevorzugt wird auf Endtemperaturen von 20 °C bis -10 °C abgekühlt, insbesondere auf Temperaturen von 10 °C bis -5 °C, ganz besonders bevorzugt auf Temperaturen von 10 °C bis 5 °C. Die Kristalle werden abgetrennt und mit Isopropanol und anschließend mit Wasser gewaschen. Das Trocknen der Substanz erfolgt bei erhöhter Temperatur bevorzugt bei 60 °C unter verminderten Druck oder bei Normaldruck.

Ein bevorzugtes Verfahren besteht darin die Verbindung der Formel I in 80 %igem Isopropanol bei der Siedetemperatur von Isopropanol unter Normaldruck oder verminderten Druck zu lösen und anschließend die heiße Lösung langsam abzukühlen, so daß die Kristallisation bei Temperaturen von mehr als 40 °C, bevorzugt von 40 °C bis 85 °C, besonders bevorzugt von 45 °C bis 80 °C, insbesondere von 50 °C bis 70 °C, erfolgt. Die ausgefallenen Kristalle werden dann mehrmals mit Isopropanol gewaschen und unter verminderten Druck getrocknet. Die Kristallisation kann ohne Animpfen mit Kristallen der Modifikation 1 erfolgen oder bevorzugt in Anwesenheit von Kristallen der Modifikation 1, die durch Animpfen in die Lösung, enthaltend die Verbindung der Formel I, eingebracht werden. Das Animpfen kann auch mehrfach bei unterschiedlichen Temperaturen erfolgen. Die Menge des Animpfmaterials hängt von der Menge der Lösung ab und kann von einem Fachmann leicht ermittelt werden.

Ein besonders bevorzugtes Verfahren zur Herstellung der Verbindung der Formel I in der Modifikation 1 besteht darin, daß man
a) die Verbindung der Formel I, die nicht in der Modifikation 1 vorliegt oder Mischungen der Modifikation 1 und anderer Kristallformen der Verbindung der Formel I in ein organisches Lösungsmittel oder in Mischungen von organischen Lösungsmittel und Wasser überführt,
b) die erhaltene Mischung auf eine Temperatur von 41°C bis zur Siedetemperatur des organischen Lösungsmittels erwärmt,
c) die erhaltene Lösung mit Wasser verdünnt oder das organische Lösungsmittel abdestilliert, so daß das organische Lösungsmittel und das Wasser in einem Verhältnis von 4:1 bis 0,3:1 vorliegen und
d) die Kristallisation bei Temperaturen oberhalb von 40°C durchführt.

Bevorzugt wird die erhaltene Lösung nach Verfahrensschritt b) filtriert.

Durch das besonders bevorzugte Verfahren können auch Mischungen, enthaltend die Modifikation 1 und 2, gezielt in die Modifikation 1 überführt werden. Dazu werden Kristalle der Modifikation 2 oder Mischungen von Modifikation 1 und 2 in einer Mischung, enthaltend organische Lösungsmittel und Wasser gelöst. Als Lösungsmittel geeignet sind beispielsweise mit Wasser mischbare Lösungsmittel wie (C₁-C₄)-Alkohole, z.B. Methanol, Ethanol, Propanol, Isopropanol, Butanol oder Isobutanol, aber auch Ketone wie Aceton oder Methylethylketon.

Vorteilhafte Mischungen enthalten organisches Lösungsmittel und Wasser im Verhältnis von 1:1 bis 8:1, bevorzugt von 2:1 bis 6:1, insbesondere von 3:1 bis 5:1.

Die Herstellung der Lösung erfolgt vorzugsweise bei erhöhter Temperatur, insbesondere bei Temperaturen von 41°C bis zum Siedepunkt des jeweiligen Lösungsmittels. Die erwärmte Lösung wird beispielsweise für einige Zeit auf Siedetemperatur gehalten um eine vollständige Lösung der Verbindung der Formel I sicherzustellen. Der Lösevorgang kann auch bei erhöhtem Druck durchgeführt werden. Danach wird die Lösung filtriert. Der eingesetzte Filter hat einen Porendurchmesser von etwa 0,1 µm bis 200 µm. Der filtrierten Lösung wird anschließend Wasser zugegeben, das vorteilhaft die gleiche Temperatur wie die filtrierte Lösung hat, oder das organische Lösungsmittel wird abdestilliert. Die erhaltenen Lösungen enthalten vorteilhaft das organische Lösungsmittel und Wasser im Verhältnis von 4:1 bis 0,3 :1, bevorzugt von 2:1 bis 0,6:1, insbesondere bevorzugt von 1,6:1 bis 0,8:1. Danach wird langsam abgekühlt bis zu einer minimalen Temperatur von 40°C. Die Kristalle werden abgetrennt und mit Isopropanol und anschließend mit Wasser gewaschen. Das Trocknen der Substanz erfolgt vorteilhaft bei erhöhter Temperatur bevorzugt bei 60 °C unter verminderten Druck oder bei Normaldruck.

Ein insbesondere bevorzugtes Verfahren besteht darin die Verbindung der Formel I in einer Mischung von Isopropanol und Wasser im Verhältnis von 4:1 bis 5:1 und be Siedetemperatur von Isopropanol unter Normaldruck oder verminderten Druck zu lösen und zu filtrieren. Anschließend wird heiße Lösung mit soviel gleichwarmem Wasser versetzt, daß ein Verhältnis von Isopropanol zu Wasser von 2:1 bis 0,8:1 vorliegt. Danach erfolgt die Kristallisation bei Temperaturen von mehr als 40 °C, bevorzugt von 40 °C bis 85 °C, besonders bevorzugt von 45 °C bis 80 °C, insbesondere von 50 °C bis 70°C. Die ausgefallenen Kristalle werden dann mehrmals mit Isopropanol gewaschen und unter verminderten Druck getrocknet.

Ein weiteres Verfahren zur Herstellung der Modifikation 1 aus der Modifikation 2 oder einer Mischung, enthaltend die Modifikation 1 und 2, besteht darin, daß die Kristalle erhitzt werden auf eine Temperatur von über 40 °C bis 130 °C, bevorzugt von 50 °C bis 110 °C, insbesondere von 70 °C bis 105 °C, ganz besonders bevorzugt 100 °C. Die Überführung der Modifikation 2 in 1 ist von der Temperatur abhängig und dauert beispielsweise bei 100 °C von 2 bis 5 Stunden, bevorzugt von 2 bis 3 Stunden.

Ein weiteres Verfahren zur Herstellung der Modifikation 1 besteht in der Herstellung einer Suspension, enthaltend Kristalle der Modifikation 2 oder eine Mischung von Kristallen enthaltend die Modifikation 1 und 2, und einem Lösungsmittel. Die Modifikation 1 der Verbindung der Formel I erhält man durch Erwärmen der Suspension der Kristalle in einem Lösungsmittel auf eine Temperatur von mehr als 40 °C, bevorzugt 41 °C bis 100 °C, insbesondere von 50 °C bis 70 °C. Die Herstellung ist im wesentlichen von der Temperatur abhängig. Vorteilhaft sind Lösemittel, in denen sich die Verbindung der Formel I schlecht löst. Beispielsweise kann Wasser verwendet werden oder wäßrige Lösungen mit (C₁-C₄)-Alkoholen und/oder mit Ketonen wie Methylethylketon oder Aceton. In der Regel erfolgt das Erwärmen in wäßriger Suspension, zweckmäßigerweise unter Rühren oder Schütteln. Die Temperaturbehandlung erfolgt so lange, bis die Modifikation 2 vollständig in die Modifikation 1 überführt ist.

Die vollständige Überführung der Modifikation 2 in die Modifikation 1 ist von der Temperatur abhängig und dauert in der Regel bei einer Temperatur von 50 °C von 20 Stunden bis 28 Stunden, bevorzugt 24 Stunden. Die Kontrolle der Reaktion erfolgt röntgenographisch oder IR-spektroskopisch mittels während der Behandlung entnommener Proben.

### Beispiel 1

### Herstellung der Modifikation 1

Wasserfeuchtes Leflunomid, wird roh zunächst in Isopropanol/Wasser gelöst (entsprechend 16 kg Leflunomid, roh, trocken, in 28 I Isopropanol plus die Menge Wasser, die zusammen mit dem Wassergehalt des feuchten Produktes eine Gesamt-Wassermenge von 9 I ergibt).

Anschließend wird auf 78°C bis 82°C erwärmt, 25 Minuten (min) bei dieser Temperatur gerührt und dann über einen Drucktrichter in einen ebenfalls schon auf gleiche Temperatur geheizten Kessel filtriert. Der Drucktrichter wird mit der Menge Isopropanol nachgespült, die zusammen mit eingesetztem Isopropanol (iPrOH) ein Verhältnis von iPrOH/Wasser von 4:1 ergeben (hier 4 l). Danach wird ebenfalls auf 78°C bis 82°C vorgeheiztes Wasser zugesetzt (32 I, ergibt iPrOH/Wasser = 0,8:1). Dabei trübt sich die Lösung bereits, die dann in 20 min auf etwa 65°C gekühlt, etwa 40 min bei dieser Temperatur gehalten, danach in 70 min auf etwa 40°C gekühlt und noch 20 min nachgerührt wird. Das Produkt wird durch Zentrifugation isoliert.

Tabelle 1 zeigt die Ergebnisse von 4 Ansätzen.

**Tabelle 1:**

| Ansatz | Ausgangs-Konzentration | Verhältnis iPrOH/H₂O | Endkonzentration | Anteil* Kristalle Modifikation 2 | Ausbeute |
|---|---|---|---|---|---|
| | [g/l] | | [g/l] | [%] | [%] |
| 1 | 600 | 4:1 | 600 | n.b. | 73,2 |
| 2 | 600 | 3:1 | 563 | <0,4 | 71,4 |
| 3 | 400 | 2:1 | 333 | <0,4 | 70,5 |
| 4 | 400 | 0,8:1 | 222 | <0,4 | 85,6 |

| | | | | | |
|---|---|---|---|---|---|
| * Die Bestimmung erfolgte durch Röntgen-Pulverdiffraktometrie; der Anteil der Modifikation 2 war immer unterhalb der Nachweisgrenze, die bei etwa 0,4 % liegt. n.b. bedeutet nicht bestimmt | | | | | |

## Patentansprüche

1. Verfahren zur Herstellung der Modifikation 1 der Verbindung der Formel I, 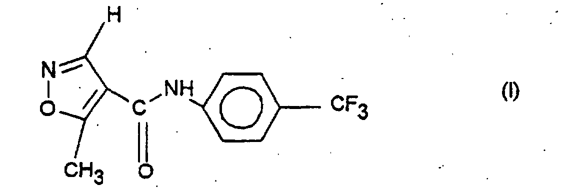 die im Röntgenbeugungsdiagramm in Transmission mit fokusierendem Debye-Scherrer-Strahlengang und Cu-K_{α1}-Strahlung Linien bei folgenden Beugungswinkeln 2 Theta (°) aufweist:
Linien starker Intensität: 16,70; 18,90; 23,00; 23,65; 29,05
Linien mittlerer Intensität: 8,35; 12,65; 15,00; 15,30; 18,35; 21,25; 22,15; 24,10; 24,65; 25,45; 26,65; 27,40; 28,00; 28,30;
**dadurch gekennzeichnet, daß** die Verbindung der Formel I, die nicht in der Modifikation 1 vorliegt oder Mischungen, enthaltend die Modifikationen 1 und Modifikation 2 der Verbindung der Formel I, die im Röntgenbeugungsdiagramm in Transmission mit fokusierendem Debye-Scherrer-Strahlengang und Cu-K_{α1}-Strahlung Linien bei folgenden Beugungswinkeln 2 Theta (°) aufweist:
Linien starker Intensität: 10,65; 14,20; 14,80; 16,10;21,70; 23,15; 24,40; 24,85; 25,50; 25,85; 26,90; 29,85
Linien mittlerer Intensität: 7,40; 9,80; 13,10; 15,45; 16,80; 20,70; 21,45; 22,80; 23,85; 27,25; 28,95,
in einem organischen Lösemittel oder Mischungen von organischem Lösemittel und Wasser gelöst wird und bei Temperaturen von mehr als 40 °C, bevorzugt von 41 °C bis 80 °C, insbesondere von 50 °C bis 70 °C, kristallisiert wird.

2. Verfahren gemäß Anspruch 1, **dadurch gekennzeichnet, daß** man
a) die Verbindung der Formel I, die nicht in der Modifikation 1 vorliegt oder Mischungen der Modifikation 1 und anderer Kristallformen der Verbindung der Formel I in ein organisches Lösungsmittel oder in Mischungen von organischem Lösungsmittel und Wasser überführt,
b) die erhaltene Mischung auf eine Temperatur von 41 °C bis zur Siedetemperatur des organischen Lösungsmittels erwärmt,
c) die erhaltene Lösung mit Wasser verdünnt oder das organische Lösungsmittel abdestilliert, so daß das organische Lösungsmittel und das Wasser in einem Verhältnis von 4: bis 0,3: 1 vorliegen und
d) die Kristallisation bei Temperaturen oberhalb von 40 °C durchführt.

3. Verfahren gemäß Anspruch 2, **dadurch gekennzeichnet, daß** die erhaltene Lösung nach Verfahrensschritt b) filtriert wird.

4. Verfahren gemäß einem oder mehreren der Ansprüche 1 bis 3, **dadurch gekennzeichnet, daß** als organisches Lösungsmittel Methanol, Ethanol, Propanol, Isopropanol, Butanol, Isobutanol, Aceton, Methylethylketon oder Mischungen derselben eingesetzt werden.

5. Verfahren gemäß einem oder mehreren der Ansprüche 2 bis 4, **dadurch gekennzeichnet, daß** die Mischung aus organischem Lösungsmittel und Wasser gemäß Verfahrensschritt b) auf eine Temperatur von 40 °C bis 85 °C erwärmt wird.

6. Verfahren gemäß einem oder mehreren der Ansprüche 2 bis 4, **dadurch gekennzeichnet, daß** das Verhältnis von organischem Lösungsmittel zu Wasser bei Verfahrensschritt a) von 1 : 1 bis 8 : 1, bevorzugt von 2 : 1 bis 6 : 1, insbesondere von 3 : 1bis 5 : 1, beträgt.

7. Verfahren gemäß einem oder mehreren der Ansprüche 3 bis 6, **dadurch gekennzeichnet, daß** die erwärmte Mischung durch ein Filter von 0,1 µm bis 200 µm Porendurchmesser filtriert wird.

8. Verfahren gemäß einem oder mehreren der Ansprüche 2 bis 7, **dadurch gekennzeichnet, daß** das Verhältnis von organischem Lösungsmittel zu Wasser im Verfahrensschritt c) von 2 : 1 bis 0,6 : 1, bevorzugt von 1,6 : 1 bis 0,8 : 1, beträgt.

9. Verfahren gemäß einem oder mehreren der Ansprüche 1 bis 8, **dadurch gekennzeichnet, daß** bei der Kristallisation die Temperatur von 83 °C bis 85 °C auf etwas über 40 °C abgesenkt wird.

10. Verfahren gemäß Anspruch 2, **dadurch gekennzeichnet, daß** das organische Lösungsmittel Isopropanol ist, die Temperatur beim Lösen der Verbindung der Formel I 85 °C beträgt, ein Filter von 1 µm Porendurchmesser eingesetzt wird, das Verhältnis von Isopropanol und Wasser im Filtrat von 1,6 : 1 bis 0,8 : 1 beträgt und die Kristallisation beim Abkühlen von 83 °C auf etwa 41 °C erfolgt

11. Verfahren zur Herstellung der Modifikation 1 der Verbindung der Formel I gemäß Anspruch 1, **dadurch gekennzeichnet, daß** die Verbindung der Formel I, die nicht in der Modifikation 1 vorliegt oder Mischungen der Modifikation 1 und Modifikation 2 gemäß Anspruch 1, in Suspension auf eine Temperatur von mehr als 40 °C, insbesondere von 41 °C bis 100 °C, bevorzugt 50 °C bis 70 °C, erhitzt wird.

12. Verfahren gemäß Anspruch 11, **dadurch gekennzeichnet, daß** eine wäßrige Suspension vorliegt.

## Claims

1. A process for the preparation of modification 1 of the compound of the formula I 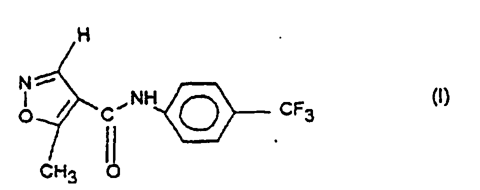 which, in the transmission X-ray diffraction pattern with focusing Debye-Scherrer beam and Cu-K_{α1} radiation, has lines at the following 2 theta angles (°):
lines of strong intensity: 16.70; 18.90; 23.00; 23.65; 29.05
lines of medium intensity: 8.35; 12.65; 15.00; 15.30;
18.35; 21.25; 22.15; 24.10;
24.65; 25.45; 26.65; 27.40;
28.00; 28.30;
wherein the compound of the formula I which is not present as modification 1 or a mixture containing the modification 1 and modification 2 of the compound of the formula I, which, in the transmission X-ray diffraction pattern with focusing Debye-Scherrer beam and Cu-K_{α1} radiation, has lines of the following 2 theta angles (°):
lines of strong intensity: 10.65; 14.20; 14.80; 16.10;
21.70; 23.15; 24.40; 24.85;
25.50; 25.85; 26.90; 29.85
lines of medium intensity: 7.40; 9.80; 13.10; 15.45;
16.80; 20.70; 21.45; 22.80;
23.85; 27.25; 28.95
is dissolved in an organic solvent or a mixture of an organic solvent and water and is crystallized at temperatures of more than 40°C, preferably from 41°C to 80°C, in particular from 50°C to 70°C.

2. The process as claimed in claim 1, wherein
a) the compound of the formula I which is not present as modification 1 or a mixture of modification 1 and other crystal forms of the compound of the formula I is transferred to an organic solvent or to a mixture of an organic solvent and water
b) the mixture obtained is heated to a temperature of from 41°C to the boiling point of the organic solvent,
c) the solution obtained is diluted with water or the organic solvent is distilled of so that the organic solvent and the water are present in a ratio of from 4:1 to 0.3:1 and
d) the crystallization is carried out at temperatures above 40°C.

3. The process as claimed in claim 2, wherein the solution obtained is filtered after process step b).

4. The process as claimed in one or more of claims 1 to 3, wherein the organic solvent used is methanol, ethanol, propanol, isopropanol, butanol, isobutanol, acetone, methyl ethyl ketone or a mixture thereof.

5. The process as claimed in one or more of claims 2 to 4, wherein the mixture of organic solvent and water is heated to a temperature of from 40°C to 85°C according to process step b).

6. The process as claimed in one or more of claims 2 to 4, wherein the ratio of organic solvent to water in process step a) is from 1:1 to 8:1, preferably from 2:1 to 6:1, in particular from 3:1 to 5:1.

7. The process as claimed in one or more of claims 3 to 6, wherein the heated mixture is filtered through a filter having a pore diameter of from 0.1 µm to 200 µm.

8. The process as claimed in one or more of claims 2 to 7, wherein the ratio of organic solvent to water in process step c) is from 2:1 to 0.6:1, preferably from 1.6:1 to 0.8:1.

9. The process as claimed in one or more of claims 1 to 8, wherein, during the crystallization, the temperature is reduced from 83°C to 85°C to slightly above 40°C.

10. The process as claimed in claim 2, wherein the organic solvent is isopropanol, the temperature during dissolution of the compound of the formula I is 85°C, a filter having a pore diameter of 1 µm is used, the ratio of isopropanol to water in the filtrate is from 1.6:1 to 0.8:1 and the crystallization is carried out during cooling from 83°C to about 41°C.

11. The process for the preparation of modification 1 of the compound of formula I as claimed in claim 1, wherein the compound of the formula I which is not present as modification 1 or a mixture of modification 1 and modification 2 as claimed in claim 1 is heated in suspension to a temperature of more than 40°C, in particular from 41°C to 100°C, preferably from 50°C to 70°C.

12. The process as claimed in claim 11, wherein an aqueous suspension is present.

## Revendications

1. Procédé pour la préparation de la forme 1 du composé de formule I 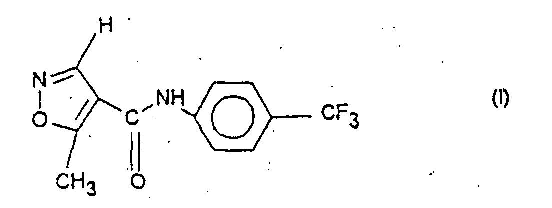 qui présente dans le diagramme de diffraction des rayons X en transmission sous une trajectoire d'un faisceau avec la méthode de Debye-Scherrer focalisante et avec la radiation K_{α1} du Cu, des raies aux angles de diffraction 2θ (°) suivants:
raies de forte intensité : 16,70; 18,90; 23,00; 23,65; 29,05;
raies d'intensité moyenne: 8,35; 12,65; 15,00; 15,30; 18,35; 21,25; 22,15;
24,10; 24,65; 25,45; 26,65; 27,40; 28,00;
28,30;
**caractérisé en ce qu'**on dissout le composé de formule I, qui ne se trouve pas sous la forme 1, ou des mélanges contenant la forme 1 et la forme 2 du composé de formule I, qui présente dans le diagramme de diffraction des rayons X en transmission sous une trajectoire d'un faisceau avec la méthode de Debye-Scherrer focalisante et avec la radiation K_{α1} du Cu, des raies aux angles de diffraction 2θ (°) suivants:
raies de forte intensité : 10,65; 14,20; 14,80; 16,10; 21,70; 23,15; 24,40;
24,85; 25,50; 25,85; 26,90; 29,85;
raies d'intensité moyenne : 7,40; 9,80; 13,10; 15,45; 16,80: 20,70; 21,45;
22,80; 23,85; 27,25; 28,95;
dans un solvant organique ou des mélanges de solvant organique et d'eau, et on le fait cristalliser à des températures de plus de 40°C, de préférence de 41°C à 80°C, en particulier de 50°C à 70°C.

2. Procédé selon la revendication 1, **caractérisé en ce que**
a) on transfère le composé de formule I, qui ne se trouve pas sous la forme 1, ou des mélanges de la forme 1 et d'autres formes cristallines du composé de formule I, dans un solvant organique ou dans des mélanges de solvant organique et d'eau,
b) on chauffe le mélange obtenu à une température de 41°C au point d'ébullition du solvant organique,
c) on dilue avec de l'eau la solution obtenue ou on élimine par distillation le solvant organique, de manière que le solvant organique et l'eau se trouvent en un rapport de 4:1 à 0,3:1, et
d) on effectue la cristallisation à des températures supérieures à 40°C.

3. Procédé selon la revendication 2, **caractérisé en ce que** la solution obtenue est filtrée après l'étape b) du procédé.

4. Procédé selon une ou plusieurs des revendications 1 à 3, **caractérisé en ce qu'**on utilise comme solvant organique le méthanol, l'éthanol, le propanol, l'isopropanol, le butanol, l'isobutanol, l'acétone, la méthyléthylcétone ou des mélanges de ceux-ci.

5. Procédé selon une ou plusieurs des revendications 2 à 4, **caractérisé en ce que** le mélange de solvants organiques et d'eau selon l'étape b) du procédé est chauffé à une température de 40°C à 85°C.

6. Procédé selon une ou plusieurs des revendications 2 à 4, **caractérisé en ce que** le rapport du solvant organique à l'eau dans l'étape a) du procédé va de 1:1 à 8:1, de préférence de 2:1 à 6:1, en particulier de 3:1 à 5:1.

7. Procédé selon une ou plusieurs des revendications 3 à 6, **caractérisé en ce que** le mélange chauffé est filtré à travers un filtre ayant un diamètre de pores de 0,1 µm à 200 µm.

8. Procédé selon une ou plusieurs des revendications 2 à 7, **caractérisé en ce que** le rapport du solvant organique à l'eau dans l'étape c) du procédé va de 2:1 à 0,6:1, de préférence de 1,6:1 à 0,8:1.

9. Procédé selon une ou plusieurs des revendications 1 à 8, **caractérisé en ce que**, lors de la cristallisation, on abaisse la température, de 83-85°C, à légèrement au-dessus de 40°C.

10. Procédé selon une la revendication 2, **caractérisé en ce que** le solvant organique est l'isopropanol, la température lors de la dissolution du composé de formule I est de 85°C, on utilise un filtre ayant un diamètre de pores de 1 µm, le rapport de l'isopropanol à l'eau dans le filtrat va de 1,6:1 à 0,8:1 et la cristallisation s'effectue lors du refroidissement, de 83°C, à environ 41°C.

11. Procédé pour la préparation de la forme 1 du composé de formule I selon la revendication 1, **caractérisé en ce qu'**on chauffe le composé de formule I, qui ne se trouve pas sous la forme 1, ou des mélanges de la forme 1 et la forme 2 selon la revendication 1, en suspension, à une température de plus de 40°C, en particulier de 41°C à 100°C, de préférence de 50°C à 70°C.

12. Procédé selon la revendication 11, **caractérisé en ce qu'**une suspension aqueuse est présente.
